(19) Europäisches Patentamt
European Patent Office
Office européen des brevets

(11) Publication number: **0 399 464 A2**

(12) # EUROPEAN PATENT APPLICATION

(21) Application number: 90109698.2

(22) Date of filing: 22.05.90

(51) Int. Cl.5: G01N 33/68, G01N 33/574, G01N 33/92, G01N 33/543

(30) Priority: 24.05.89 JP 132285/89

(43) Date of publication of application: 28.11.90 Bulletin 90/48

(84) Designated Contracting States: BE CH DE FR GB IT LI SE

(71) Applicant: Ishikawa, Eiji
24-1, Ohtsukadainishi 3-chome
Miyazaki-shi Miyazaki 880-21(JP)

Applicant: SUMITOMO PHARMACEUTICALS COMPANY, LIMITED
2-8, Doshomachi 2-chome, Chuo-ku
Osaka-shi Osaka 541(JP)

(72) Inventor: Ishikawa, Eiji
24-1 Ohtsukadainishi 3-chome
Miyazaki-shi Miyazaki 880-21(JP)
Inventor: Katumaru, Hiroyuki
15-10-105, Kusunoki-cho
Ashiya-shi, Hyogo 659(JP)

(74) Representative: Myerscough, Philip Boyd et al
J.A. Kemp & Co. 14 South Square, Gray's Inn
GB-London WC1R 5LX(GB)

(54) **Assay method for a substance with a specific sugar chain.**

(57) An assay method for a substance with a specific sugar chain, comprising;

(A) adding a functional-group-bound antibody which recognizes a substance with a specific sugar chain to a sample solution containing the substance, followed by binding a complex comprising the substance and the antibody to a carrier via the functional group,

(B) selectively dissociating the complex comprising the substance and the antibody from the carrier,

(C) binding the substance and a lectin which recognizes the specific sugar chain thereof together, and

(D) determining the amount of the complex comprising the lectin, the substance and the antibody is described. According to the present invention, a glycoprotein with a specific sugar chain can be assayed simply and with high sensitivity without any influence from the coexisting substances in the sample.

EP 0 399 464 A2

# Assay Method for a Substance with a Specific Sugar Chain

## BACKGROUND OF THE INVENTION

The present invention relates to an assay method for a substance with a specific sugar chain.

Assay of proteins such as hormones and antibodies is diagnostically very important, and most of these proteins are glycoproteins with a sugar chain. In recent years, progress in analytical techniques for sugar chains has revealed that these glycoproteins involve different types of glycoproteins which have different sugar chain structures even when their protein moiety is the same. In addition, it has been found that a particular glycoprotein with a specific sugar chain solely increases in some diseases. For example, a type of alpha fetoprotein (AFP) with a sugar chain reactive to lentil lectin (LCA) increases in serum from patients with hepatocellular carcinoma, and another type of AFP which is weakly reactive to LCA appears in yolk sac tumor. Also, similar phenomena have been reported in $\alpha_1$-acid glycoprotein, alkaline phosphatase (ALP) and human chorionic gonadotropin (hCG) [Kazuhisa Takeda: Journal of Clinical Pathology, suppl., No. 79, p. 139 (1988)]. With this background, it is desired that an assay method permitting the recognition of the specificity of a sugar chain in diagnosis of cancer, hepatitis and other diseases be developed.

Presently, trace glycoproteins as above are assayed quantitatively by immunoassay using a specific antibody. This method, however, is unsatisfactory as to specificity as an assay method for the target glycoprotein with a specific sugar chain because it uses an antibody which recognizes protein moieties due to the absence of an antibody which differentiates as far as sugar chains of glycoproteins.

In the meantime, lectin has long been known as a substance which specifically recognizes a particular sugar chain. Some attempts have been made to identify diseases, particularly tumors, by differentiating specific sugar chain structures, including the attempts to differentiate primary hepatoma and yolk sac tumor by identifying the sugar chain structure of alpha fetoprotein which appears in serum, reported in "Scandinavian Journal of Immunology", vol. 14, p. 15 (1981) and "SRL HOKAN", vol. 12, No. 3, p. 30 (1988). The identification method used is lectin adsorption crossed immunoelectrophoresis in the former and affinity chromatography using lectin-bound Sepharose in the latter. However, these methods both involve complicated procedures, and neither of which is a simple and quick assay method which can be used for diagnosis in clinical situations.

In view of this aspect, an easily-manipulated sandwich assay method using lectin and antibody in combination has recently been proposed. In this method, 1) an antibody-bound solid phase is reacted with a sample to trap the target glycoprotein, 2) labeled lectin is added to form a sandwich- like complex, and 3) the enzyme-labeled complex is measured [Kinoshita et al.: Clinica Chimica Acta, vol. 179, p. 143 (1989)].

This method permits quick and simple assay, but the use of lectin-sugar chain coupling poses some problems other than those posed in the case of protein-recognizing antibody for the reasons shown below. That is, in many cases, the specific sugar chain of the target glycoprotein with a specific sugar chain is present in a large amount in another glycoprotein coexisting in the sample. For example, AFP, a glycoprotein, is known to involve two types having different sugar chain structures, one of which is produeced by cancerous cells, while the other is produced by normal cells. Lectin which recognizes the sugar chain of AFP produced by cancerous cells also recognizes the sugar chain of IgG which is present in a large amount in the sample. Therefore, in reacting an antibody-bound solid phase and a sample to bind the target glycoprotein, a background effect occurs due to the non-specifically bound IgG. Such background effect varies among samples, thus posing a problem in assay reliability.

It is possible to solve this problem by the method in which a labeled antibody is added after reaction of a lectin-bound solid phase and a sample, but this method requires a large volume of solid phase, resulting in increase of the background effect on the labeled antibody. Thus, satisfactory reliability cannot be expected.

As stated above, conventional assay methods for substances with a specific sugar chain are unsatisfactory either in specificity, operativity or reliability. A high-sensitivity sugar chain assay technique for clinical use which meets all these requirements remains to be developed in the relevant technical field.

## SUMMARY OF THE INVENTION

The object of the present invention is to provide a new assay method which permits simple and highly

sensitive measurement of a glyco protein with a specific sugar chain without influence from the coexisting substances in the sample.

The present invention is an assay method for a substance with a specific sugar chain comprising;

(A) adding a functional-group-bound antibody which recognizes a substance with a specific sugar chain to a sample solution containing the substance, followed by binding a complex comprising the substance and the antibody to a carrier via the functional group,

(B) selectively dissociating the complex comprising the substance and the antibody from the carrier,

(C) binding the substance and a lectin which recognizes the specific sugar chain thereof together, and

(D) determining the amount of the complex comprising the lectin, the substance and the antibody.

## BRIEF DESCRIPTION OF THE DRAWINGS

Figs. 1, 2, 3 and 4 respectively show the results ob tained in Examples 1, 2, 3 and 4 according to the present invention, and those obtained in Comparison Examples 1, 2, 3 and 4 according to a conventional method. The abscissa indicates the amount of hAFP added, and the ordinate indicates the fluorescence intensity for bound β-D-galactosidase activity.

## DETAILED DESCRIPTION OF THE INVENTION

The present invention is hereinafter described in detail in the order of processes.

(A)

This is a process in which 1) a specific antibody against the substance with a specific sugar chain is added to a sample solution containing the substance to form an immunocomplex and 2) the immunocomplex is bound to a carrier via a functional group.

Examples of sample solutions favorable for the embodiment of the present invention include various body fluids such as blood, cellular tissue fluid, lymph, pleural effusion, ascites, amniotic fluid, gastric juice, urine, pancreatic juice, cerebrospinal fluid and saliva, with preference given to blood, particularly serum or blood plasma. Examples of substances with a specific sugar chain include glycoproteins and glycolipids contained in these body fluids.

Examples of glycoproteins include γ-glutamyl transpeptitase (γ-GTP), α-fetoprotein (AFP), carcinoembryonic antigen (CEA), alkaline phosphatase (ALP), amylase (AMY) and human chorionic gonadotropin (HCG). Examples of glycolipids include gangliosides $GM_1$, $GM_2$, $GM_3$, $GD_{1a}$, $GD_2$ and $GD_3$, Forssman antigen and globoside.

The methods for preparing the antibodies against these substances with a specific sugar chain are known or in accordance with a known method.

The antibody against the substance with a specific sugar chain needs to have been bound with the functional group involved in the coupling of the complex with the carrier in (A). The functional group means, for example, one partner of a specific binding system selected from among hapten-hapten antibody, biotin-avidin (streptoavidin) or antigen-antibody, which is to be bound with a target substance containing a sugar chain, and examples thereof include hapten such as dinitrophenyl group and trinitrophenyl group, biotin, antibody or antigen. Coupling of the functional group can be carried out by a known method.

The carrier is not subject to any limitation, and any carrier used for conventional immunoassay methods can be used, as long as it does not interfere with the object of the present invention. Examples of such carrier include polystyrene, polyacryl, Teflon, paper, glass and agarose. There is also no limitation posed on carrier form. The carrier needs to have a reactive group for the coupling of the complex formed in (A) or for the formation of a complex on the carrier. Herein, the reactive group is another partner in a specific binding system, which is supplemental to the functional group and which is to be bound with a carrier.

Examples of the reactive group bound to the carrier include ordinary reactive groups corresponding to the functional group, for example:

1) an antibody against the functional group when it is a hapten of dinitrophenyl group or trinitrophenyl

group,

2) avidin or streptoavidin when the functional group is a biotin, and

3) an antibody or antigen corresponding to the funtional group when it is an antigen or antibody.

If necessary, the carrier is bound to the complex by forming a bond which permits dissociation of the complex from the carrier without complex decomposition, such as -S-S-bond of the functional group and the reactive group.

Coupling of the reactive group to the carrier can be achieved by a known method.

In the present invention, the complex comprising a substance with a specific sugar chain and a functional-group-bound antibody is normally bound to the carrier by either of the following methods (a) and (b) chosen as appropriate.

(a) A complex comprising a substance with a specific sugar chain in the sample solution and a functional-group-bound antibody is formed and then bound to a carrier.

(b) The complex is formed on a carrier.

Complex formation in the above-mentioned method (a) is carried out, for example, under conditions used for ordianry antigen-antibody reaction in the presence of a functional-group-bound antibody added to the sample solution. The complex is formed normally at 0 to 45 °C for several to scores of hours, preferably 20 to 37°C for 1 to 6 hours. Thereafter, a carrier having a reactive group is added to the sample solution and the complex is bound to the carrier under the same conditions as above.

The above-mentioned method (b) is carried out by simultaneous addition of a functional-group-bound antibody and a reactive-group-bound carrier to the sample solution. The reaction is carried out under the same conditions as in method (a). The complex is formed normally at 0 to 45 °C for several to scores of hours, preferably 20 to 37 °C for 1 to 6 hours.

After the above-mentioned complex is bound to the carrier, the carrier is normally washed under conditions used for ordinary immunoassay methods.

<u>(B)</u>

This is a process of selective dissociation from the carrier of the complex which has been bound thereto, comprising a substance with specific sugar chain, a specific antibody against the substance and other components. A preferable method for selective dissociation of the above-mentioned complex from the carrier is such that does not break immunological bound of the complex. Generally, this is achieved by addition to the sample solution of a sufficient amount of a substance having the same reaction site as with the functional group used to bind the complex and the carrier. For example, when the functional group is a dinitrophenyl group, dinitrophenylamino acid (e. g. dinitrophenyl-lysine) is added; when the functional group is a biotin, biotin is added.

When the binding is linked by -S-S- bond, a reducing agent (e.g. 2-mercaptoethanol) is added.

<u>(C)</u>

This is a process for reacting the substance with a specific sugar chain, with a lectin which recognizes the specific sugar chain to form a sugar chain-lectin bond.

Any lectin can be selected from known animal- or plant-derived lectins. Examples of lectins include ConA (Jack bean lectin), RCA120 (castor bean lectin, castor bean agglutinin), RCA60 (castor bean lectin, lysine), LCA (lentil lectin), PSA (pea lectin), VFA (broad bean lectin), L-PHA (kidney bean lectin), E-PHA (kidney bean lectin), WGA (wheat germ lectin), PNA (peanut lectin), agalactoprotein-bound lectin (avian derivation), asialoprotein-bound lectin (mammalian derivation), sarcolectin (derived from <u>Sarcophaga peregrina</u>), AAL (<u>Aleuria aurantia</u>) and DSA (<u>Datura Stamonium</u>). These lectins recognize different sugar chain structures, and a suitable one can be selected according to the subject and purpose of assay.

The lectin can be used in various forms, including 1) direct addition to the sample solution, 2) use after immobilization to a carrier (another carrier) other than the carrier used in (A) by a known method of protein immobilization, 3) use with previous coupling with the marker substance in (D), and 4) use with previous coupling with a functional group for binding with another reactive-group-bound carrier or a reactive-group-bound marker.

Also, (C) can be carried out before, after or simultaneously with (A) and (B), and any mode can be employed as appropriate according to assay conditions.

More specifically, the above-mentioned complex comprising a sugar chain-lectin bond is labeled by a

method such as 1) a lectin-immobilizing carrier is used to form the complex on another carrier, after which a marker antibody is added to label the complex, or 2) a labeled lectin is added to the complex which has been bound to an antibody-immobilizing carrier, comprising a substance with a specific sugar chain and an antibody; thereafter, the labeled complex is assayed quantitatively in the next (D).

Reaction of the lectin and the substance with a specific sugar chain is carried out by a known method.

(D)

This is a process of determination of the amount of the sandwich-like complex comprising a lectin, a substance with a specific sugar chain and an antibody, formed in (A), (B) and (C), on the basis of the marker bound to the complex.

Any substance can serve as the marker, as long as it is usable for immunoassay. Examples of such substance include enzymes, radioactive substances, fluorescent substances, luminescent substances and metal compounds.

Examples of usable enzymes include peroxidase, β-D-galactosidase and alkaline phosphatase. Examples of usable radioactive substances include $^{3}H$, $^{125}I$, $^{131}I$, $^{14}C$ and $^{32}P$. Examples of usable fluorescent substances include fluorescein isothiocyanate. Examples of usable luminescent substances include acridium salts. Examples of usable metal compounds include europium.

Any method of marker binding can be used, as long as it has conventionally been used to bind a marker to an antibody or antigen in immunoassay methods.

The complex bound onto the carrier can be measuered by a known method.

As stated above, it is possible to establish an appropriate assay system for the detection and quantitative assay of a substance with a specific sugar chain in body fluids samples, which has been difficult to do, by various modes in combination of formations of an antigen-antibody complex and a sugar-chain-lectin bond carried out in (A), (B) and (C).

Some representative applications are hereinafter described by means of the following symbols:

| | Symbol |
|---|---|
| Substance with a specific sugar chain (s represents a specific sugar chain) | : X-s |
| Functional group | : f |
| Reactive group | : r |
| Marker | : m |
| Lectin | : L |
| Specific antibody against X | : [X], [X]′ |
| Specific antibody against [X] | : [[X]] |
| Specific antibody against L | : [L] |
| Carrier | : C |

Affinity bond is represented by =; covalent bond or adsorption bond is represented by -.

Type I: Lectin solid phase type

1. [X]-f is added to the sample solution containing X-s to form immunocomplex f-[X]=X-s.
2. C-r is added to form C-r=f-[X]=X-s, followed by washing.
3. An excess amount of f is added to dissociate f-[X]=X-s, followed by removal of C-r=f.
4. C-L is added to form C-L=s-X=[X]-f, followed by washing.
5. [X]′-m is added to form

$$\begin{array}{c} C-L=s-\underset{\begin{array}{c}\| \\ [X]'-m\end{array}}{X}=[X]-f. \end{array}$$

6. m is used to assay the complex obtained in 5 quantitatively.

Type II: Labeled lectin type 1

1. [X]-f is added to the sample solution containing X-s to form immunocomplex f-[X]=X-s.
2. C-r is added to form C-r=f-[X]=X-s, followed by washing.
3. L-m is added to form C-r=f-[X]=X-s=L-m, followed by washing.
4. An excess amount of f is added to dissociate f-[X]=X-s=L-m, followed by removal of C-r=f.
5. C-[[X]] is added to form $C\text{-}[[X]] = [\underset{\overset{|}{f}}{X}] = X\text{-}s = L\text{-}m$, followed by washing.
6. m is used to assay the complex obtained in 5 quantitatively.

Type III: Labeled lectin type 2

1. [X]-f is added to the sample solution containing X-s to form immunocomplex f-[X]=X-s.
2. C-r is added to form C-r=f-[X]=X-s, followed by washing.
3. An excess amount of f is added to dissociate f-[X]=X-s, followed by removal of C-r=f.
4. L-m is added to form f-[X]=X-s=L-m.
5. C-[[X]] is added to form $C\text{-}[[X]] = [\underset{\overset{|}{f}}{X}] = X\text{-}s = L\text{-}m$, followed by washing.
6. m is used to assay the complex obtained in 5 quantitatively.

Type IV: Lectin single addition type 1

1. [X]-f is added to the sample solution containing X-s to form immunocomplex f-[X]=X-s.
2. L is added to form f-[ X ] =X-s=L.
3. C-r is added to form C-r=f-[X]=X-s=L, followed by washing.
4. An excess amount of f is added to dissociate f-[X]=X-s=L, followed by removal of C-r=f.
5. C-[[X]] is added to form $C\text{-}[[X]] = [\ X\ ] = \underset{\overset{|}{f}}{X}\text{-}s = L$, followed by washing.
6. [L]-m is added to form $C\text{-}[[X]] = [\ X\ ] = \underset{\overset{|}{f}}{X}\text{-}s = L = [L]\text{-}m$, followed by washing.
7. m is used to assay the complex obtained in 6 quantitatively.

Type V: Lectin single addition type 2

1. [X]-f is added to the sample solution containing X-s to form immunocomplex f-[X]=X-s.
2. C-r is added to form C-r=f-[X]=X-s, followed by washing.
3. L is added to form C-r=f-[X]=X-s=L, followed by washing.
4. [L]-m is added to form C-r=f-[X]=X-s=L=[L]-m, followed by washing.
5. An excess amount of f is added to dissociate f-[X]=X-s=L=[L]-m, followed by removal of C-r=f.
6. C-[[X]] is added to form $C\text{-}[[X]] = [\underset{\overset{|}{f}}{X}] = X\text{-}s = L = [L]\text{-}m$, followed by washing.
7. m is used to assay the complex obtained in 6 quantitatively.

Type I is advantageous in that excellent specificity and high sensitivity are obtained since it is free of reduction in the ability of the lectin solid phase to bind with the subject substance caused by coexisting substances having the same specific sugar chain binding to the lectin solid phase.

Types II and III are advantageous in that excellent specificity and high sensitivity are obtained since they are free of increase in background effect caused by coexisting substances having the same specific sugar chain non-specifically binding to the antibody solid phase.

Type IV and V are advantageous in that excellent specificity and high sensitivity are obtained since it is free of increase in background effect caused by the lectin used in a large amount non-specifically binding to the antibody solid phase.

The present invention is hereinafter described in more detail by means of the following examples, but the invention is not by any means limited to them.

Example 1

1. Preparation of affinity-purified dinitrophenyl-goat anti-human alpha fetoprotein (hAFP) IgG

(1) Preparation of mercaptosuccinyl-goat anti-AFP IgG

Thiol groups were introduced into goat anti-AFP IgG in accordance with a known method [Ishikawa et al.: Journal of Immunoassay, 4, 209 (1983)] using S-acetylmercaptosuccinic anhydride (Nakalai Tesque, Kyoto). The number of thiol groups introduced was 9.7 per goat anti-AFP IgG molecule.

(2) Preparation of maleimide-dinitrophenyl-L-lysine

2.0 ml of a 0.1 M sodium phosphate buffer containing 5.5 mM dinitrophenyl-L-lysine hydrochloride (Tokyo Kasei, Tokyo), pH 7.0, and 0.2 ml of N,N-dimethylformamide containing 5.5 mM N-succinimidyl-6-maleimide hexanoate were reacted at 30 °C for 30 minutes.

(3) Preparation of dinitrophenyl-goat anti-hAFP IgG

1.21 ml of the maleimide-dinitrophenyl-L-lysine prepared in (2) and 5.0 mg of the mercaptosuccinyl-goat anti-hAFP IgG prepared in (1) in solution in 5.2 ml of a 0.1 M sodium phosphate buffer, pH 6.0, containing 5 mM EDTA, were reacted at 30 °C for 30 minutes. After completion of the reaction, gel filtration was conducted using Sephadex G-25 (Pharmacia, Sweden) to yield dinitrophenyl-goat anti-hAFP IgG, wherein the column size was 1.0 x 30 cm and a 0.1 M sodium phosphate buffer, pH 6.0, containing 5 mM EDTA, was used as eluent.

The number of dinitrophenyl groups introduced was 4.2 per mercaptosuccinyl-goat anti-hAFP IgG molecule, as calculated from the absorbance at 360 nm for a molar extinction coefficient of 17400 $mol^{-1} \cdot \ell \cdot cm^{-1}$.

(4) Preparation of affinity-purified dinitrophenyl-goat anti-hAFP IgG

The dinitrophenyl-goat anti-hAFP IgG was affinity-purified by a known method in which elution is conducted with hydrochloric acid of pH 2.5 [Kohno et al.: Journal of Biochemistry, 100, 1247 (1986)] using an hAFP insolubilized Sepharose 4B column.

2. Purification of lentil lectin bound hAFP

0.5 ml of serum of primary hepatoma patients was passed through a lentil lectin-Sepharose 4B column with a size of 1.0 x 20 cm (Pharmacia) equilibrated with a 0.1 M sodium acetate buffer, pH 7.0, containing 0.5 M NaCl, 1 mM $MgCl_2$, 1 mM $CaCl_2$ and 1 mM $MnCl_2$, followed by column washing with about 60 ml of the same buffer. The hAFP adsorbed to the lentil lectin column was eluted with the same buffer containing 1 M glucose to purify the lentil lectin bound hAFP.

The lentil lectin bound hAFP was subjected to ultra-filtration for concentration and buffer exchange and then dissolved in a solution of 0.1% BSA, 1 mM $CaCl_2$, 1 mM $MgCl_2$, 1 mM $MnCl_2$ and 0.005% thimerosal in a 10 mm Tris buffer, pH 7.0.

3. Preparation of affinity-purified rabbit

Anti(dinitrophenyl-bovine serum albumin) IgG insolubilized solid phase

Rabbit anti(dinitrophenyl-bovine serum albumin) IgG was affinity-purified by a known method in which elution is conducted with hydrochloric acid of pH 2.5 [Kohno et al.: Journal of Biochemistry, mentioned above] using a dinitrophenyl- bovine serum albumin insolubilized Sepharose 4B column.

Using a solution of the affinity-purified rabbit anti-(dinitrophenyl-bovine albumin) IgG (0.1 g/l), the IgG was physically adsorbed onto the surface of polystyrene balls (3.2 mm in diameter, produced by Precision Plastic Ball Co., Chicago) by a known method [Ishikawa et al.: Scandinavian Journal of Immunology, 8 (suppl. 7), 43 (1978)] to prepare an affinity-purified rabbit anti(dinitrophenyl-bovine serum albumin) IgG insolubilized solid phase.

4. Preparation of lentil lectin insolubilized solid phase

Using a lentil lectin solution (0.1 g/ℓ), lentil lectin was physically adsorbed onto the surface of polystyrene balls (3.2 mm in diameter, produced by Precision Plastic Ball Co., Chicago) by a known method [Ishikawa et al.: Scandinavian Journal of Immunology, mentioned above] to prepare a lentil lectin insolubilized solid phase.

5. Preparation of affinity-purified anti-hAFP Fab′-β-D-galactosidase

(1) Preparation of affinity-purified goat anti-hAFP $F(ab')_2$

Goat anti-hAFP IgG was digested by a known method using pepsin [Ishikawa et al.: Scandinavian Journal of Immunology, mentioned above] to prepare its $F(ab')_2'$ followed by affinity-purification by a known method in which elution is conducted with hydrochloric acid of pH 2.5 [Kohno et al.: Journal of Biochemistry, mentioned above] using an hAFP insolubilized Sepharose 4B column to prepare affinity-purified goat anti- hAFP $F(ab')_2$.

(2) Preparation of affinity-purified goat anti-hAFP Fab′-β-D-galactosidase

The affinity-purified goat anti-hAFP $F(ab')_2$ was labeled with galactosidase by a known method [Ishikawa et al.: Scandinavian Journal of Immunology, mentioned above] using N-N′-O-phenylenedimaleimide as crosslinking agent.

1.7 units of Fab′ were bound to each β-D-galactosidase molecule.

The affinity-purified goat anti-hAFP Fab′-β-D-galactosidase was passed through a lentil lectin-Sepharose 4B column (Pharmacia) with a size of 1.0 x 7 cm.

6. Preparation of dinitrophenyl-non-specific rabbit Fab′

(1) Preparation of mercaptosuccinyl-non-specific rabbit Fab′

Non specific rabbit IgG was digested by a known method using pepsin [Ishikawa et al.: Scandinavian Journal of Immunology, mentioned above] to prepare non-specific rabbit $F(ab')_2$. S-acetylmercapto groups were introduced into this non-specific rabbit $F(ab')_2$ in accordance with a known method [Ishikawa et al.: Journal of Immunoassay (mentioned above)] using S-acetylmercaptosuccinic anhydride (Nakalai Tesque, Kyoto). Hydroxylamine and mercaptoethylamine were used to obtain non-specific rabbit Fab′ with thiol groups introduced. The number of thiol groups introduced was 5.6 per non-specific rabbit Fab′ molecule.

(2) Preparation of maleimide-dinitrophenyl-L-lysine

2.0 ml of N,N-dimethylformamide containing 100 mM dinitrophenyl-L-lysine hydrochloride (Tokyo Kasei, Tokyo) and 6 ml of a 0.1 M sodium phosphate buffer, pH 7.0, containing 5 mM EDTA, were mixed together, followed by reaction with 0.8 ml of N,N-dimethylformamide containing 55 mM N-succinimidyl-6-maleimide

hexanoate at 30 °C for 30 minutes.

(3) Preparation of dinitrophenyl-non-specific rabbit Fab′

8.8 ml of the maleimide-dinitrophenyl-L-lysine prepared in (2) and 60 mg of the mercaptosuccinyl-non-specific rabbit Fab′ prepared in (1) in solution in a 0.1 M sodium phosphate buffer, pH 6.0, containing 5 mM EDTA, were reacted at 30 °C for 30 minutes. After completion of the reaction, gel filtration was conducted using Ultrogel AcA44 (LKB, Sweden) to yield dinitrophenyl-non-specific rabbit Fab′, wherein the column size was 5 x 45 cm and a 10 mM Tris buffer, pH 7.0, containing 0.1 M NaCl, 1 mM $MgCl_2$, 1 mM $CaCl_2$, 1 mM $MnCl_2$, 0.1% BSA and 0.1% $NaN_3$, was used as eluent.

The number of dinitrophenyl groups introduced was 3.7 per mercaptosuccinyl-non-specific rabbit Fab′ molecule.

7. Determination of β-D-galactosidase activity

β-D-galactosidase activity was determined by a known fluorometric method [Imagawa et al.: Analytial Biochemistry, 20, 310 (1984)] after reaction using 4-methylumbelliferyl-β-D-galactoside as substrate at 30 °C for 30 minutes. Fluorescence intensity was determined using $10^{-7}$ M 4-methylumbelliferone in solution in a 0.1 M glycine-NaOH buffer, pH 10.3, as the reference.

8. Assay of lentil lectin bound hAFP

A sample containing 100 fmol affinity-purified dinitrophenyl-goat anti-hAFP IgG, hAFP and serum was dissolved in 150 μℓ of a 10 mM sodium phosphate buffer, pH 7.0, containing 0.4 M NaCl, 0.1% BSA and 0.1% $NaN_3$, followed by reaction at 20 °C for 3 hours.

Two balls of the affinity-purified rabbit anti(dinitrophenyl-bovine serum albumin) IgG insolubilized solid phase was added, followed by reaction at 20 °C for 3 hours.

After washing solid phase twice by addition of 2 ml of an ice-cooled 10 mM sodium phosphate buffer, pH 7.0, containing 0.1 M NaCl, 0.1% BSA and 0.1% $NaN_3$ (Buffer A), 130 μℓ of a 1 mM dinitrophenyl-lysine solution in a 10 mM Tris buffer, pH 7.0, containing 0.1 M NaCl, 1 mM $MgCl_2$, 1 mM $CaCl_2$, 1 mM $MnCl_2$, 0.1% BSA and 0.1% $NaN_3$ (Buffer B) was added, followed by reaction at 20 °C for 1 hour. The affinity-purified rabbit anti(dinitrophenyl-bovine serum albumin) IgG insolubilized solid phase was removed, and the reaction mixture was kept standing at 4 °C overnight.

20 μℓ of a solution of 75 μg of dinitrophenyl-non-specific rabbit Fab′ in Buffer B and then two balls of the lentil lectin insolubilized solid phase were added, followed by reaction at 20 °C for 3 hours.

After washing solid phase twice by addition of 2 ml of Buffer B, a solution of 10 fmol of affinity-purified goat anti-hAFP Fab′-β-D-galactosidase in 150 μℓ Buffer B was added, followed by reaction at 20 °C for 3 hours.

After washing solid phase twice by addition of 2 ml of ice-cooled Buffer B, the solid-phase-bound β-D-galactosidase activity was determined at 30 °C for 150 minutes.

The results are shown in Fig. 1.

## Comparison Example 1

The procedure of Example 1 was followed to prepare a lentil lectin insolubilized solid phase and affinity-purified goat anti-hAFP Fab′-9-D-galactosidase.

1. Assay of lentil lectin bound hAFP

A sample containing hAFP and serum was dissolved in 150 μℓ of a 10 mM Tris buffer, pH 7.0, containing 0.4 M NaCl, 1 mM $MgCl_2$, 1 mM $CaCl_2$, 1 mM $MnCl_2$, 0.1% BSA and 0.1% $NaN_3$, followed by addition of two balls of the lentil lectin insolubilized solid phase and reaction at 20 °C for 3 hours and then

at 4° C overnight.

After washing solid phase twice by addition of 2 ml of an ice-cooled 10 mM Tris buffer, pH 7.0, containing 0.1 M NaCl, 1 mM $MgCl_2$, 1 mM $CaCl_2$, 1 mM $MnCl_2$, 0.1% BSA and 0.1% $NaN_3$ (Buffer B), a solution of 10 fmol of the affinity-purified goat anti-hAFP Fab′-β-D-galactosidase in 150 μℓ of Buffer B was added, followed by reaction at 20° C for 3 hours.

After washing solid phase twice by addition of 2 ml of ice-cooled Buffer B, the solid-phase-bound β-D-galactosidase activity was determined at 30° C for 30 minutes.

As shown in Fig. 1, hAFP could be assayed quantitatively at a high sensitivity (3 fmol tube), whether serum was present or not, in Example 1 according to the present invention. However, noticiable sensitivity reduction occurred in the presence of serum in Comparison Example 1 according to a conventional method.

## Example 2

The procedure of Example 1 was followed to prepare affinity-purified dinitrophenyl-goat anti-human alpha feto-protein (hAFP) IgG and an affinity-purified rabbit anti(dinitrophenyl-bovine serum albumin) IgG insolubilized solid phase and purify lentil lectin bound hAFP.

### 1. Preparation of lentil lectin-β-D-galactosidase

#### (1) Preparation of mercaptosuccinyl-lentil lectin

Thiol groups were introduced into lentil lectin in accordance with a known method [Ishikawa et al.: Journal of Immunoassay, mentioned above] using S-acetylmercaptosuccinic anhydride (Nacalai Tesque, Kyoto). The number of thiol groups introduced was 0.7 per lentil lectin molecule.

#### (2) Preparation of maleimide-β-D-galactosidase

Maleimide groups were introduced into β-D-galactosidase by a known method [Ishikawa et al.: Scandinavian Journal of Immunology, mentioned above] using N,N′-o-phenylenedimaleimide. The number of maleimide groups introduced was 12 per β-D-galactosidase molecule.

#### (3) Preparation of lentil lectin-β-D-galactosidase

15 ml of a 0.1 M sodium phosphate buffer, pH 6.0, containing 0.67 mg of the mercaptosuccinyl-lentil lectin prepared in (1) and a solution of 1.07 mg of the maleimide-β-D-galactosidase prepared in (2) in 1.26 ml of a 0.1 M sodium phosphate buffer, pH 6.0, were mixed together. This mixture was concentrated to 1 ml by ultrafiltration, followed by reaction at 4° C overnight. After completion of the reaction, gel filtration was conducted using a Sepharose CL-6B column to yield lentil lectin-β-D-galactosidase, wherein the column size was 1.5 x 65 cm and a 10 mM sodium phosphate buffer, pH 7.0, containing 0.01% BSA, 0.1 M NaCl, 1mM $MgCl_2$ and 0.1% $NaN_3$ was used as eluent. The number of lentil lectin units introduced was 2.7 per β-D-galactosidase molecule.

### 2. Affinity-purified rabbit anti(goat IgG) $F(ab')_2$ insolubilized solid phase

Rabbit anti(goat IgG) IgG was digested with pepsin in the same manner as Example 1-1 and then purified using a normal goat IgG insolubilized Sepharose 4B column to yield affinity-purified rabbit anti(goat IgG) $F(ab')_2$. This affinity-purified rabbit anti(goat IgG) $F(ab')_2$ was physically adsorbed onto polystyrene balls in the same manner as in Example 1-3 to yield an affinity-purified rabbit anti( goat IgG) $F(ab')_2$ insolubilized solid phase.

3. Assay of lentil lectin bound hAFP

A sample containing 100 fmol of affinity-purified dinitrophenyl-goat anti-hAFP IgG, hAFP and serum was dissolved in 150 μℓ of a 10 mM sodium phosphate buffer, pH 7.0, containing 0.4 M NaCl, 0.1% BSA and 0.1% $NaN_3$, followed by reaction at 20° C for 3 hours.

Two balls of the affinity-purified rabbit anti(dinitrophenyl-bovine serum albumin) IgG insolubilized solid phase were added, followed by reaction at 20° C for 3 hours and then at 4° C overnight.

After washing solid phase twice by addition of 2 ml of an ice-cooled 10 mM sodium phosphate buffer, pH 7.0, containing 0.1 M NaCl, 0.1% BSA and 0.1% $NaN_3$ (Buffer A), a solution of 1 pmol of lentil lectin-β-D-galactosidase in a 10 mM Tris buffer, pH 7.0, containing 0.1 M NaCl, 1 mM $MgCl_2$, 1 mM $CaCl_2$, 1 mM $MnCl_2$, 0.1% BSA and 0.1% $NaN_3$ (Buffer B), was added, followed by reaction at 20° C for 3 hours.

After washing solid phase twice by addition of 2 ml of ice-cooled Buffer B, 150 μℓ of a 1 mM dinitrophenyl-lysine solution in Buffer B was added, followed by reaction at 20° C for 1 hour. After removal of the affinity-purified rabbit anti(dinitrophenyl-bovine serum albumin) IgG insolubilized solid phase, two balls of the affinity-purified rabbit anti-(goat IgG) $F(ab')_2$ insolubilized solid phase were added, followed by reaction at 20° C for 3 hours. After washing solid phase twice by addition of 2 ml of ice-cooled Buffer B, the β-D-galactosidase activity bound to the solid-phase was determined.

The results are shown in Fig. 2.

## Comparison Example 2

The procedure of Example 2 were followed to prepare lentil lectin-β-D-galactosidase.

1. Preparation of goat anti-hAFP $F(ab')_2$ insolubilized solid phase

Goat anti-hAFP IgG was digested by a known method using pepsin [Ishikawa et al.: Journal of Immunoassay, mentioned above] to prepare $F(ab')_2$ which was then physically adsorbed onto the surface of polystyrene balls having a diameter of 3.2 mm by a known method [Ishikawa et al.: Scandinavian Journal of Immunology, mentioned above] using a goat anti-hAFP $F(ab')_2$ solution (0.1 g/ℓ) to prepare a goat anti-hAFP $F(ab')_2$ insolubilized solid phase.

2. Assay of lentil lectin bound hAFP

A sample containing hAFP and serum was dissolved in 150 μℓ of a 10 mM sodium phosphate buffer, pH 7.0, containing 0.4 M NaCl, 0.1% BSA and 0.1% $NaN_3$, followed by addition of two balls of the goat anti-hAFP $F(ab')_2$ insolubilized solid phase and reaction at 20° C for 3 hours and then at 4° C overnight. After washing solid phase twice by addition of 2 ml of a 10 mM sodium phosphate buffer, pH 7.0, containing 0.1 M NaCl, 0.1% BSA and 0.1% $NaN_3$ (buffer A), 100 fmol of lentil lectin-β-D-galactosidase in solution in a 10 mM Tris buffer, pH 7.0, containing 0.1M NaCl,, 1 mM $MgCl_2$, 1 mM $CaCl_2$, 1 mM $MnCl_2$, 0.1% BSA and 0.1% $NaN_3$ (Buffer B), followed by reaction at 20° C for 3 hours.

After washing solid phase twice by addition of 2 ml of ice-cooled Buffer B, the β-D-galactosidase activity bound to the solid phase was determined.

As shown in Fig. 2, hAFP could by assayed quantitatively with high sensitivities (1 to 3 fmol/tube) even in the presence of serum in Example 2 according to the present invention. In contrast, a noticeable background fluorescence (about 100 fold) was noted, with a sensitivity of about one-tenth in Comparison Example 2 according to a conventional method.

## Example 3

The procedure of Example 1 was followed to prepare affinity-purified dinitrophenyl-goat anti-human alpha fetoprotein (hAFP) IgG and an affinity-purified rabbit anti(dinitrophenyl-bovine serum albumin) IgG insolubilized solid phase and purify lentil lectin bound hAFP. The procedure of Example 2 was followed to

prepare lentil lectin-β-D-galactosidase and an affinity-purified rabbit anti(goat IgG) F(ab′)$_2$ insolubilized solid phase.

1. Preparation of affinity-purified rabbit anti(dinitrophenyl-bovine serum albumin) F(ab′)$_2$ insolubilized solid phase

Rabbit anti(dinitrophenyl-bovine serum albumin) IgG was digested by a known method using pepsin [Ishikawa et al.: Journal of Immunoassay, mentioned above] to prepare its F(ab′)$_2$ which was then affinity-purified by a known method in which elution is conducted with hydrochloric acid of pH 2.5 using a dinitrophenyl-bovine serum albumin insolubilized Sepharose 4B column [Kohno et al.: Journal of Biochemistry, mentioned above].

Using a solution of the affinity-purified rabbit anti-(dinitrophenyl-bovine serum albumin) F(ab′)$_2$ (0.1 g/ℓ), affinity-purified rabbit anti(dinitrophenyl-bovine serum albumin) F(ab′)$_2$ insolubilized solid phase was prepared by physical adsorption onto polystyrene balls having a diameter of 3.2 mm [Precision Plastic Balls, Chicago] by a known method [Ishikawa et al.: Scandinavian Journal of Immunology, mentioned above].

2. Assay of lentil lectin bound hAFP

A sample containing 100 fmol of affinity-purified dinitrophenyl-goat anti-hAFP IgG and hAFP was dissolved in 150 μℓ of a 10 mM sodium phosphate buffer, pH 7.0, containing 0.1 M NaCl, 1mM $MgCl_2$, 0.1% BSA and 0.1% $NaN_3$ (Buffer A), followed by reaction at 20°C for 3 hours. Two balls of the affinity-purified rabbit anti(dinitrophenyl-bovine serum albumin) F(ab′)$_2$ insolubilized solid phase were added, followed by reaction at 20°C overnight.

After washing solid phase twice by addition of 2 ml of ice-cooled Buffer A, 130 μℓ of a solution of 1 mM dinitrophenyl-lysine in a 10 mM Tris buffer, pH 7.0, containing 0.1 M NaCl, 1mM $MgCl_2$, 1 mM $CaCl_2$, 1 mM $MnCl_2$, 0.1% BSA and 0.1% $NaN_3$ (Buffer B), was added, followed by reaction at 20°C for 1 hour. After removal of the affinity-purified rabbit anti(dinitrophenyl-bovine serum album in) F(ab′)$_2$ insolubilized solid phase, 100 fmol of lentil lectin-β-D-galactosidase in solution in 20 μℓ of Buffer B was added, followed by reaction at 20°C for 3 hours.

The affinity-purified rabbit anti(goat IgG) F(ab′)$_2$ insolubilized solid phase was placed, followed by reaction at 20°C 3 hours.

After washing solid phase twice by addition of 2 ml of ice-cooled Buffer B, the β-D-galactosidase activity bound to the solid phase was determined.

The results are shown in Fig. 3. Note that a control was established for 0 fmol of affinity-purified dinitrophenyl-goat anti-hAFP IgG.

## Comparison Example 3

The procedure of Example 3 was followed to prepare affinity-purified dinitrophenyl-goat anti-human alpha feto-protein (hAFP) IgG, an affinity-purified rabbit anti(dinitrophenyl-bovine serum albumin) F(ab′)$_2$ insolubilized solid phase and lentil lectin-β-D-galactosidase and purify lentil lectin bound hAFP.

1. Assay of lentil lectin bound hAFP

A sample containing 100 fmol of affinity-purified dinitrophenyl-goat anti-hAFP IgG and hAFP was dissolved in 150 μℓ of a 10 mM sodium phosphate buffer, pH 7.0, containing 0.1 M NaCl, 1 mM $MgCl_2$, 0.1% BSA and 0.1% $NaN_3$ (Buffer A), followed by reaction at 20°C for 3 hours. Two balls of the affinity-purified rabbit anti(dinitrophenyl-bovine serum albumin) F(ab′)$_2$ insolubilized solid phase were added, followed by reaction at 20°C for 3 hour and then at 4°C overnight.

After washing solid phase twice by addition of 2 ml of ice-cooled Buffer A, 150 μℓ of a solution of 100 fmol of lentil lectin-β-D-galactosidase in a 10 mM Tris buffer, pH 7.0, containing 0.1 M NaCl, 1 mM $MgCl_2$, 1 mM $CaCl_2$, 1 mM $MnCl_2$, 0.1% BSA and 0.1% $NaN_3$ (Buffer B), was added, followed by reaction at 20°C for 3 hours.

After washing solid phase twice by addition of 2 ml of ice-cooled Bufer B, the β-D-galactosidase activity bound to the solid phase was determined.

The results are shown in Fig. 3. Note that a control was established for 0 fmol of affinity-purified dinitrophenyl-goat anti-hAFP IgG. In comparison Example 3 according to a conventional method, the obtained value increased with hAFP concentration even in the control group, in which no antibody which recognizes the protein site of hAFP was added to the system. This phenomenon cannot be explained from the principle of the sandwich technique, in which the subject is assayed quantitatively on the basis of the lectin-sugar chain linkage and antigen-antibody binding, posing a question as to specificity and accuracy of the quantitative assay.

On the other hand, the results obtained in Example 3 clearly show that the present invention makes it possible to determine the hAFP alone recognized by lectin and specific antibody.

## Example 4

The procedure of Example 1 was followed to prepare lentil lectin bound hAFP and an affinity-purified rabbit anti(dinitrophenyl-bovine serum albumin) IgG insolubilized solid phase.

Affinity purified dinitrophenyl-rabbit anti-human alpha fetoprotein (hAFP) IgG was prepared in accordance with Example 1, and an affinity-purified goat anti(rabbit IgG) $F(ab')_2$ insolubilized solid phase was prepared in accordance with Example 2.

### 1. Preparation of rabbit anti-lentil lectin (LCA) Fab'-β-D-galactosidase

#### (1) Preparation of rabbit anti-LCA $F(ab')_2$

Rabbit anti-LCA IgG was digested by a known method using pepsin [Ishikawa et al.: Journal of Immunoassay, mentioned above] to prepare its $F(ab')_2$.

#### (2) Preparation of rabbit anti-LCA Fab'-β-D-galactosidase

Rabbit anti-LCA $F(ab')_2$ was labeled with galactosidase by a known method using N,N'-o-phenylenedimaleimide as a crosslinking agent [Ishikawa et al.: Scandinavian Journal of Immunology, mentioned above].

### 2. Assay of lentil lectin bound hAFP

A sample containing 100 fmol of affinity-purified dinitrophenyl-rabbit anti-hAFP IgG, hAFP and serum was dissolved in 150 μℓ of a 10 mM sodium phosphate buffer, pH 7.0, con taining 0.4 M NaCl, 0.1% BSA and 0.1% $NaN_3$, followed by reaction at 20° C for 3 hours.

Two balls of the affinity-purified rabbit anti(dinitrophenyl-bovine serum albumin) IgG insolubilized solid phase were added, followed by reaction at 20° C for 3 hours and then at 4° C overnight.

After washing solid phase twice by addition of 2 ml of an ice-cooled 10 mM Tris buffer, pH 7.0, containing 0.1 M NaCl, 1 mM $MgCl_2$ 1 mM $CaCl_2$, 1 mM $MnCl_2$ and 0.1% BSA (Buffer A), 150 μℓ of a solution of 6.67 μM LCA in Buffer A was added, followed by reaction at room temperature overnight. After washing solid phase twice by addition of 2 ml of ice-cooled Buffer A, a solution of 10 fmol of rabbit anti-LCA Fab'-β-D-galactosidase in 150 μℓ of Buffer A, followed by reaction at 20° C for 3 hours. After washing solid phase twice by addition of 2 ml of ice-cooled Buffer A, 150 μℓ of a solution of 1 mM dinitrophenyl-lysine in Buffer A was added, followed by reaction at 20° C for 1 hour. After removal of the affinity-purified rabbit anti(dinitrophenyl-bovine serum albumin) IgG insolubilized solid phase, two balls of the afinity-purified goat anti(rabbit IgG) $F(ab')_2$ insolubilized solid phase were added, followed by reaction at 20° C for 3 hours. After washing solid phase twice by addition of 2 ml of ice-cooled Buffer A, the β-D-galactosidase activity bound to the solid phase was determined. The results are shown in Fig. 4.

Comparison Example 4

The procedure of Example 4 was followed to prepared affinity-purified dinitrophenyl-rabbit anti-human alpha feto-protein (hAFP) IgG, an affinity-purified rabbit anti(dinitrophenyl-bovine serum albumin) IgG insolubilized solid phase and anti-LCA Fab′-β-D-galactosidase and purify lentil lectin bound hAFP.

1. Assay of lentil lectin bound hAFP

A sample containing 100 fmol of affinity-purified dinitrophenyl-rabbit anti-hAFP IgG, hAFP and serum was dissolved in 150 µℓ of a 10 mM sodium phosphate buffer, pH 7.0, containing 0.4 M NaCl, 0.1% BSA and 0.1% $NaN_3$, followed by rection at 20° C for 3 hours.

Two balls of the affinity-purified rabbit anti(dinitrophenyl-bovine serum albumin) IgG insolubilized solid phase were added, followed by reaction at 20° C for 3 hours and then at 4° C overnight.

After washing solid phase twice by addition of 2 ml of an ice-cooled 10 mM Tris buffer, pH 7.0, containing 0.1 M NaCl, 1 mM $MgCl_2$, 1 mM $CaCl_2$, 1 mM $MnCl_2$ and 0.1% BSA (Buffer A), 150 µℓ of a solution of 6.67 µM LCA in Buffer A was added, followed by reaction at room temperature overnight. After washing solid phase twice by addition of 2 ml of ice-cooled Buffer A, a solution of 10 fmol of rabbit anti-LCA Fab′-β-D-galactosidase in 150 µℓ of Buffer A was added, followed by reaction at 20° C for 3 hours. After washing solid phase twice by addition of 2 ml of ice-cooled Buffer A, the β-D-galactosidase activity bound to the solid phase was determined. The results are shown in Fig. 4.

As shown in Fig. 4, hAFP could be quantitatively assayed with a sensitivity of up to 10 fmol/tube in Example 4 according to the present invention. However, in Comparison Example 4, a noticeable background increase occurred and hAFP could be quantitatively assayed with a sensitivity of 100 fmol/tube.

As stated above, the present invention provides a high-sensitivity, specific and quick assay method which has been unobtainable by a conventional measuring method for substances with a sugar chain in body fluids using lectin, and is expected to contribute to the development of reagents for clinical laboratories.

## Claims

1. An assay method for a substance with a specific sugar chain, comprising;

(A) adding a functional-group-bound antibody which recognizes a substance with a specific sugar chain to a sample solution containing the substance, followed by binding a complex comprising the substance and the antibody to a carrier via the functional group,

(B) selectively dissociating the complex comprising the substance and the antibody from the carrier,

(C) binding the substance and a lectin which recognizes the specific sugar chain thereof together, and

(D) determining the amount of the complex comprising the lectin, the substance and the antibody.

2. An assay method as claimed in Claim 1, wherein a sample solution is a body fluid.

3. An assay method as claimed in Claim 1, wherein a substance with a specific sugar chain is a glycoprotein or a glycolipid.

4. An assay method as claimed in Claim 1, wherein a functional group to be bound with an antibody is selected from a group consisting of hapten, biotin, antigen and antibody.

5. An assay method as claimed in Claim 1, characterized in that a carrier is bound to a complex by reacting a reactive group bound to the carrier and a functional group bound to the complex.

6. An assay method as claimed in Claim 5, wherein the combination of a functional group and a reactive group is either of the following 1 to 3:

1) the functional group is hapten and the reactive group is an antibody against the functional group

2) the functional group is biotin and the reactive group is avidin or streptoavidin

3) the functional group is an antibody or an antigen and the reactive group is a corresponding antibody or antigen.

7. An assay method as claimed in Claim 1, wherein (A) is a process for forming a complex and then binding the complex to a carrier.

8. An assay method as claimed in Claim 1, wherein (A) is a process for forming a complex on a carrier.

9. An assay method as claimed in Claim 1, characterized in that a substance having the same reaction

site as with the functional group used to bind the complex and the carrier is added for dissociation of the complex from the carrier in (B).

10. An assay method as claimed in Claim 1, wherein the amount of the complex in (D) is assayed on the basis of the marker bound to the complex.

11. An assay method as claimed in Claim 1, according to either of the following methods I to V:

Method I:

1. [X]-f is added to the sample solution containing X-s to form immunocomplex f-[X] = X-s.
2. C-r is added to form C-r = f-[X] = X-s, followed by washing.
3. An excess amount of f is added to dissociate f-[X] = X-s, followed by removal of C-r = f.
4. C-L is added to form C-L = s-X = [X]-f, followed by washing.
5. [X]′-m is added to form

$$\begin{array}{c} C-L=s-X=[X]-f. \\ \| \\ [X]'-m \end{array}$$

6. m is used to assay the complex obtained in 5 quantitatively.

Method II:

1. [X]-f is added to the sample solution containing X-s to form immunocomplex f-[X] = X-s.
2. C-r is added to form C-r = f-[X] = X-s, followed by washing.
3. L-m is added to form C-r = f-[X] = X-s = L-m, followed by washing.
4. An excess amount of f is added to dissociate f-[X] = X-s = L-m, followed by removal of C-r = f.
5. C-[[X]] is added to form $C\text{-}[[X]] = [\underset{\underset{f}{|}}{X}] = X\text{-}s = L\text{-}m$, followed by washing.
6. m is used to assay the complex obtained in 5 quantitatively.

Method III:

1. [X]-f is added to the sample solution containing X-s to form immunocomplex f-[X] = X-s.
2. C-r is added to form C-r = f-[X] = X-s, followed by washing.
3. An excess amount of f is added to dissociate f-[X] = X-s, followed by removal of C-r = f.
4. L-m is added to form f-[X] = X-s = L-m.
5. C-[[X]] is added to form $C\text{-}[[X]] = [\underset{\underset{f}{|}}{X}] = X\text{-}s = L\text{-}m$, followed by washing.
6. m is used to assay the complex obtained in 5 quantitatively.

Method IV:

1. [X]-f is added to the sample solution containing X-s to form immunocomplex f-[X] = X-s.
2. L is added to form f-[X] = X-s = L.
3. C-r is added to form C-r = f-[X] = X-s = L, followed by washing.
4. An excess amount of f is added to dissociate f-[X] = X-s = L, followed by removal of C-r = f.
5. C-[[X]] is added to form $C\text{-}[[X]] = [\underset{\underset{f}{|}}{X}] = X\text{-}s = L$, followed by washing.
6. [L]-m is added to form $C\text{-}[[X]] = [\underset{\underset{f}{|}}{X}] = X\text{-}s = L = [L]\text{-}m$, followed by washing.
7. m is used to assay the complex obtained in 6 quantitatively.

Method V:

1. [X]-f is added to the sample solution containing X-s to form immunocomplex f-[X] = X-s.
2. C-r is added to form C-r = f-[X] = X-s, followed by washing.
3. L is added to form C-r = f-[X] = X-s = L, followed by washing.
4. [L]-m is added to form C-r = f-[X] = X-s = L = [L]-m, followed by washing.
5. An excess amount of f is added to dissociate f-[X] = X-s = L = [L]-m, followed by removal of C-r = f.
6. C-[[X]] is added to form $C\text{-}[[X]] = [\underset{\underset{f}{|}}{X}] = X\text{-}s = L = [L]\text{-}m$, followed by washing.
7. m is used to assay the complex obtained in 6 quantitatively.

In the methods described above, each symbol represents the following:
X-s : Substance with a specific sugar chain (s represents a specific sugar chain)
f : Functional group
r : Reactive group
m : Marker
L : Lectin
[X], [X]′ : Specific antibody against X
[[X]] : Specific antibody against [X]
[L] : Specific antibody against L
C : Carrier
- : Covalent bond or adsorption bond
= : Affinity bond

# FIG. 1

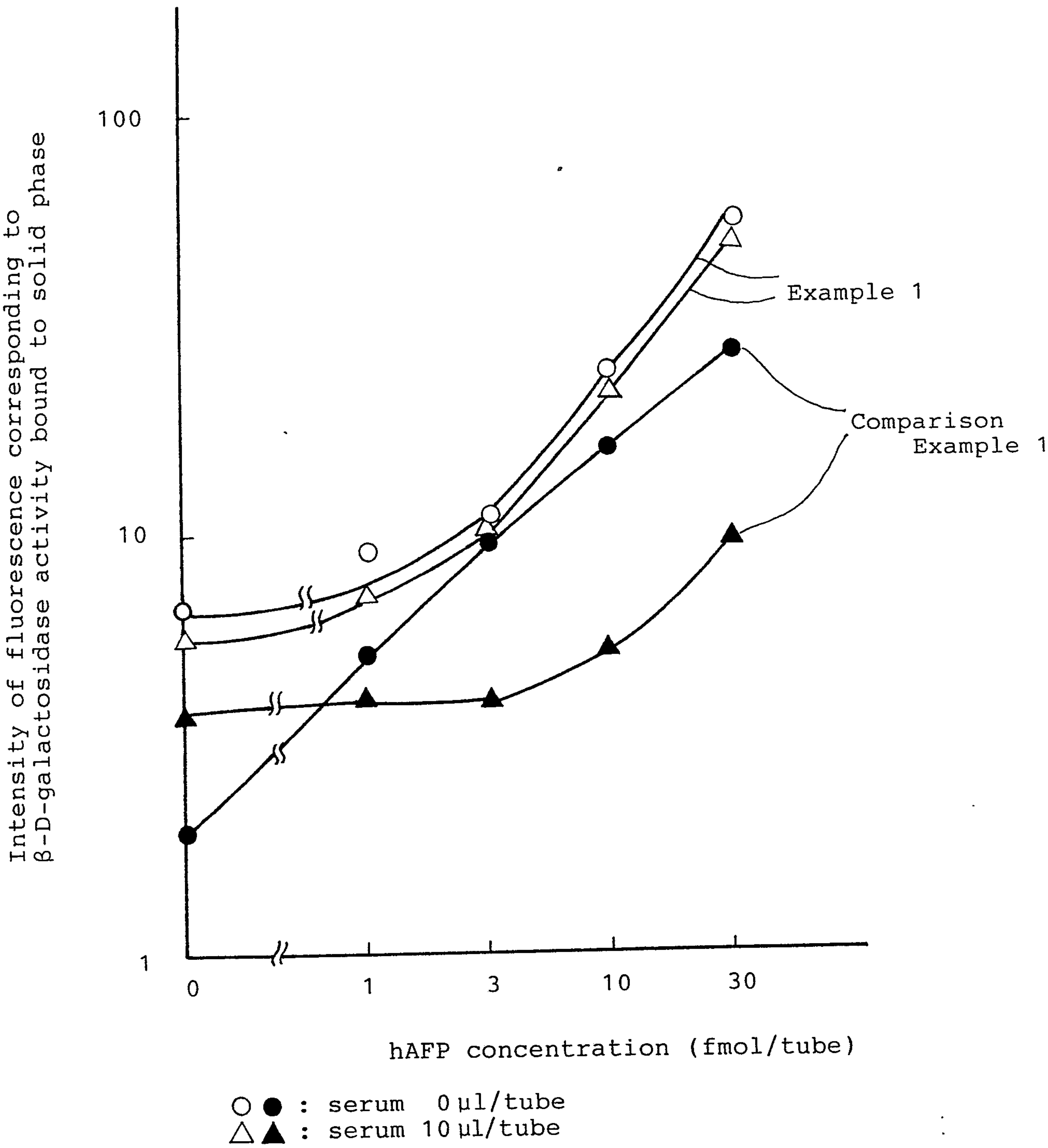
Intensity of fluorescence corresponding to β-D-galactosidase activity bound to solid phase
100
10
1
0
1
3
10
30
hAFP concentration (fmol/tube)
Example 1
Comparison Example 1
○ ● : serum 0 µl/tube
△ ▲ : serum 10 µl/tube

# FIG.2

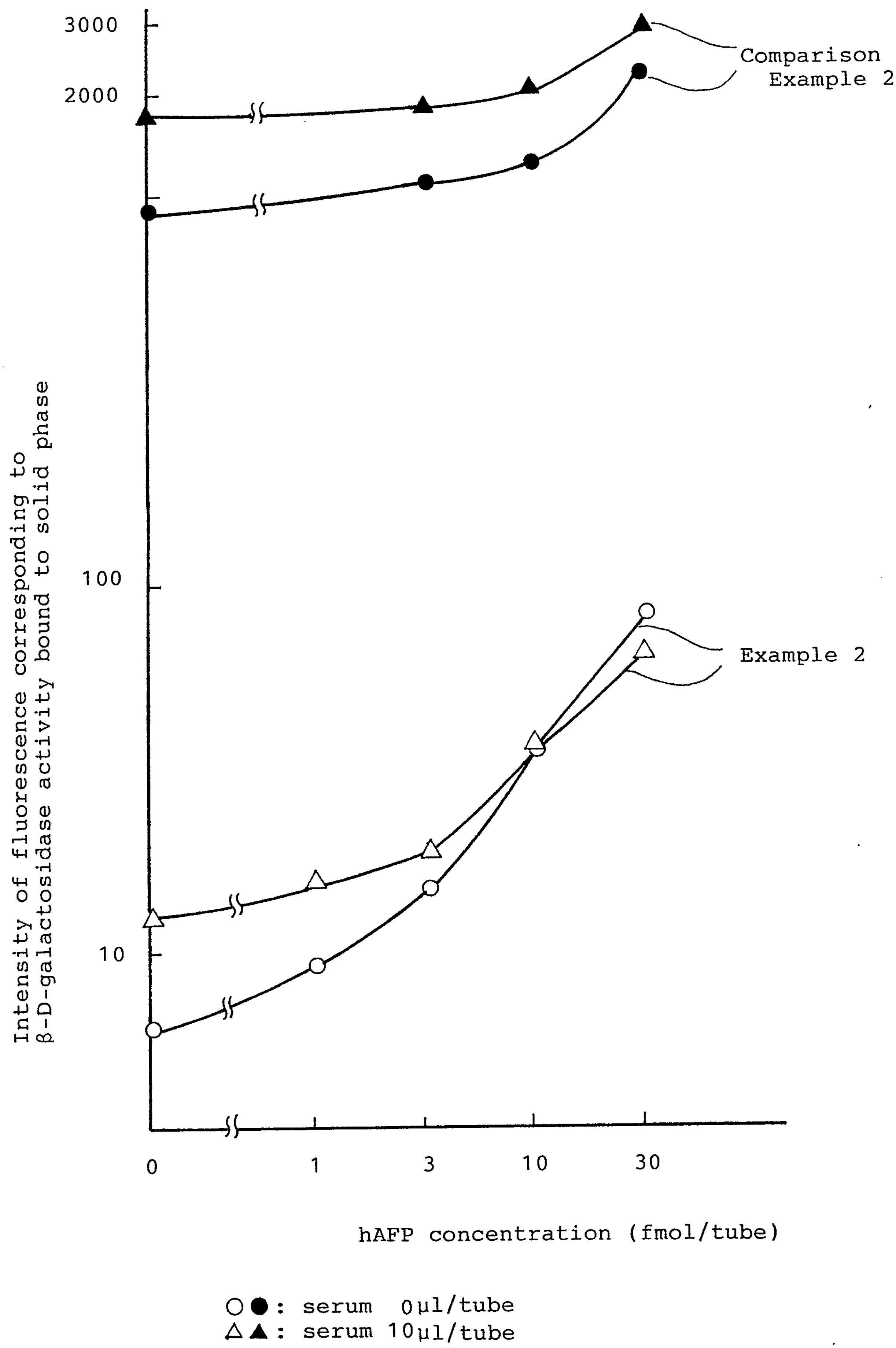
3000
2000
100
10
Comparison Example 2
Example 2
Intensity of fluorescence corresponding to β-D-galactosidase activity bound to solid phase
0
1
3
10
30
hAFP concentration (fmol/tube)
○●: serum 0μl/tube
△▲: serum 10μl/tube

# FIG.3

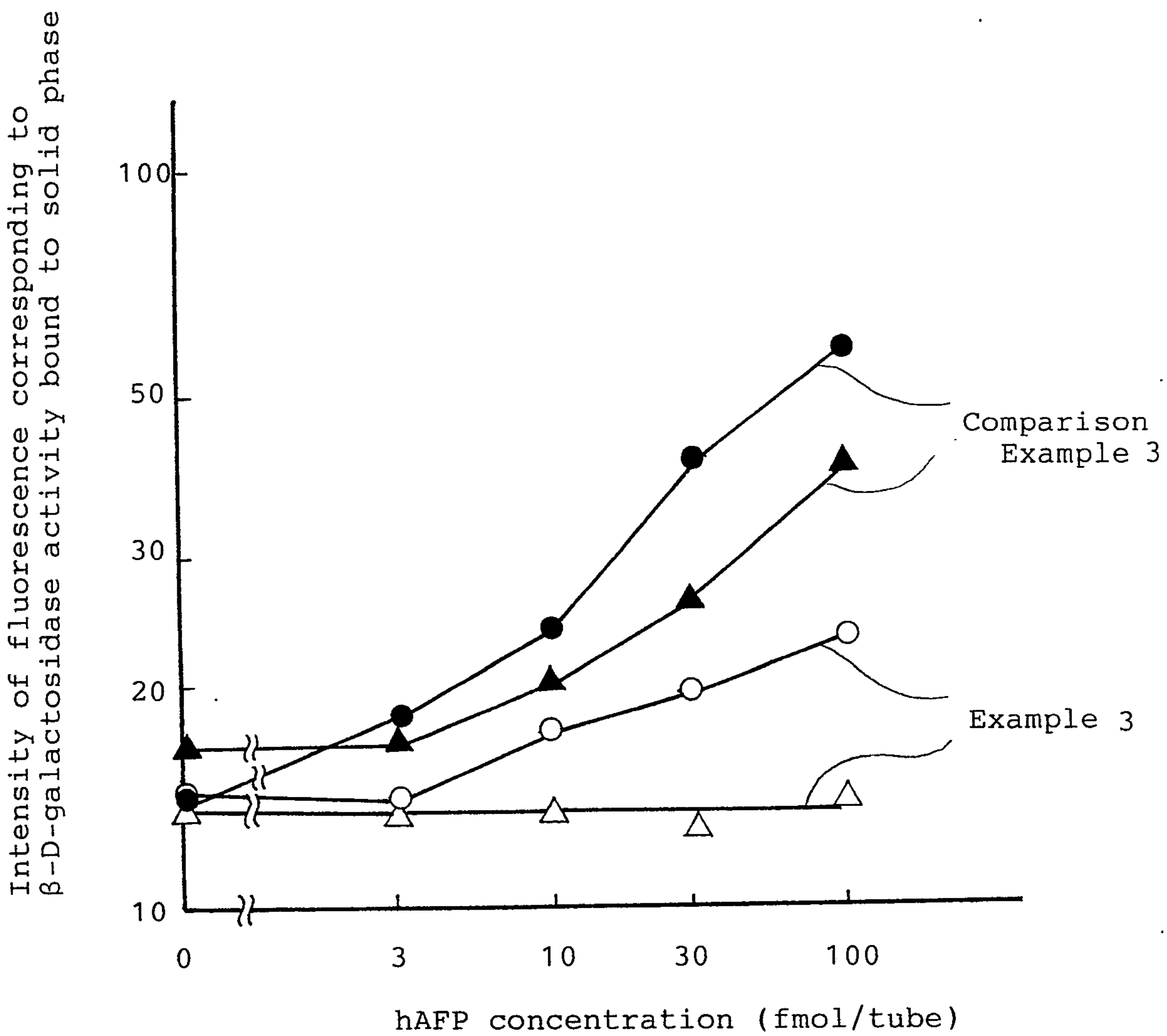


○ ● : Affinity-purified dinitrophenyl-goat antihuman AFP IgG 100 fmol/tube

△ ▲ : Affinity-purified dinitrophenyl-goat antihuman AFP IgG 0 fmol/tube

# FIG.4

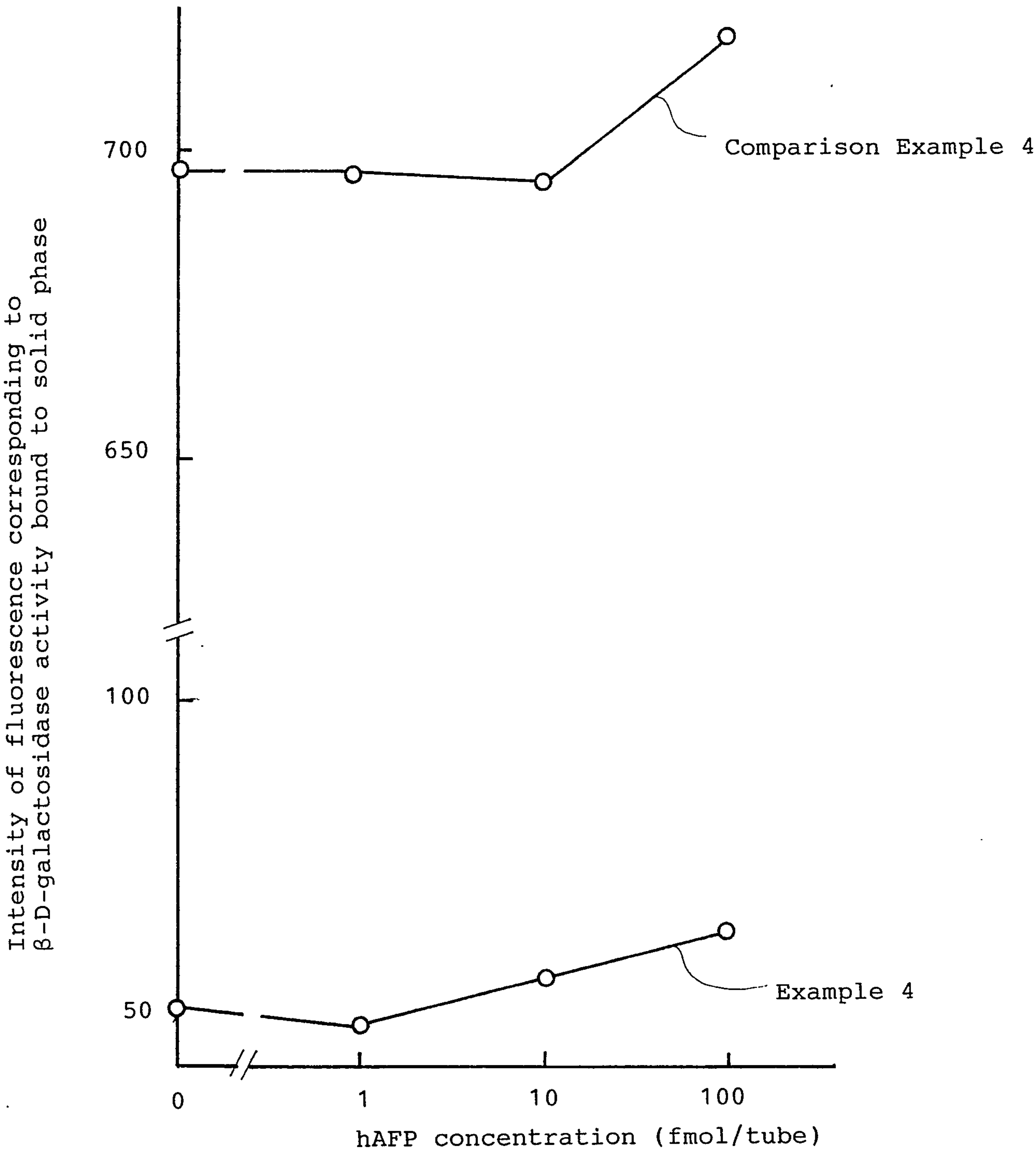
Comparison Example 4
700
650
100
50
Intensity of fluorescence corresponding to β-D-galactosidase activity bound to solid phase
Example 4
0
1
10
100
hAFP concentration (fmol/tube)